# EUROPEAN PATENT SPECIFICATION

(11) **EP 4 248 934 B1**
(45) Date of publication and mention of the grant of the patent: **01.10.2025**
(21) Application number: 21893548.4
(22) Date of filing: 15.09.2021
(51) Int. Cl.: A61H 7/00, A61F 7/00, A61H 15/00

(54) **MASSAGE HEAD ASSEMBLY**
MASSAGEKOPFANORDNUNG
ENSEMBLE TÊTE DE MASSAGE

(30) Priority: 19.11.2020 CN 202022691878 U
(43) Date of publication of application: 27.09.2023
(73) Proprietor: Xiamen Comfier Technology Co., Ltd, Xiamen, Fujian 361000 (CN)
(72) Inventor: LI, Jianqing, Xiamen, Fujian 361000 (CN); YANG, Song, Xiamen, Fujian 361000 (CN); ZHANG, Zhicheng, Xiamen, Fujian 361000 (CN)
(74) Representative: Cabinet Chaillot
(86) International application number: PCT/CN2021/118433
(87) International publication number: WO 2022/105397

(56) References cited:
- EP-A1- 2 022 460
- EP-A1- 2 272 480
- CN-A- 108 379 062
- CN-U- 203 107 628
- CN-U- 204 467 627
- CN-U- 208 958 720
- CN-Y- 201 030 026
- CN-Y- 201 030 026
- CN-Y- 201 042 497
- JP-U- 3 176 335
- US-A1- 2009 270 781

## Description

### FIELD OF THE INVENTION

The present invention relates to the technical field of massage apparatus and more particularly pertains to a massage head assembly.

### BACKGROUND OF THE INVENTION

Along with social development, there is a rapid growth of the number of brainworkers who are mostly lack of exercise and entertainment, and thus suffering from bad mood and worsening health conditions over time. Many massagers are available on the market for mood relief and body relaxation. Stimulation with a massager helps one's body to relax and provides health supporting effects.

A massager in the prior art usually comprises a massager body and a massage head assembly disposed on the massager body; the massage head assembly comprises a massage head base and a massage head disposed on the massage head base; the massage head is made of plastic materials, which provides the massage head with an overly hard texture, and leads to an uncomfortable massage experience.

EP2022460A1 discloses a heatable massage head configured for mechanical and electrical connection with a massage device; the heatable massage head has a massage-head shell, a heating means having a circuit board and a heating element electrically connecting to the circuit board, and a heating-means fixing seat; the massage-head shell and the circuit board being both fixed to the heating-means fixing seat, the massage-head shell enclosing the heating means; also including a massage-head fixing seat rotatably attached to a rotating shaft, the heating-means fixing seat being fixed to the massage-head fixing seat.

### BRIEF SUMMARY OF THE INVENTION

The present invention provides a massage head assembly with a simple structure, which is capable of providing a comfortable massage for users.

To attain the above object, the technical solutions of the present invention are as follows:

A massage head assembly, comprising a massage head base, a massage head holder and a silicone massage head; the silicone massage head is fixedly disposed on the massage head holder; the massage head holder is detachably disposed on the massage head base; the silicone massage head is of a hollow structure; the silicone massage head has an inner wall, and a buffer space is formed between the inner wall and the massage head holder; further comprising an insert; the insert is formed between a bottom part of the silicone massage head and a top surface of the massage head holder by means of secondary plastic over-molding; a plurality of positioning columns protrude from a bottom surface of the insert at intervals; a plurality of positioning holes are provided on the massage head holder; each of the positioning columns passes through the corresponding positioning hole; a fitting portion protrudes upwards from a middle part of the massage head holder; the insert is engaged with the fitting portion.

Preferably, an outer surface of the silicone massage head is covered with a cloth cover.

Preferably, the massage head assembly further comprises a heating member; the heating member is fixedly disposed inside the silicone massage head.

Preferably, the massage head assembly further comprises a light board with a plurality of light beads; the heating member is fixedly disposed on the light board; the light board is fixedly disposed on the massage head holder; the massage head holder is provided with a wire outlet for a light board electrical connection wire of the light board and a heating member electrical connection wire of the heating member to pass through.

Preferably, an accommodating column protrudes downwards from a middle part of the massage head holder; the massage head base is provided with a connection column; the connection column snaps into the accommodating column.

Preferably, at least two positioning grooves recess into an inner wall of the accommodating column at intervals; at least two positioning rods protrude from an outer wall of the connection column at intervals; each of the positioning rods is slidably fitted inside the corresponding positioning groove.

Preferably, a middle part of the massage head base is provided with an eccentric component.

By adopting the aforementioned technical solutions, the structure and design of the silicone massage heads provide the present invention with a simple overall structure; due to the good flexibility of the silicone massage head, and the buffer space formed between the inner wall of the silicone massage head and the massage head holder being capable of elastic deformation, the elasticity of the silicone massage head is increased, so that when the present invention is used to massage a human body, the silicone massage head can elastically deform to a larger degree, thereby providing a comfortable massage.

### BRIEF DESCRIPTION OF THE DRAWINGS

FIG.1 shows a perspective view of the present invention;
FIG.2 shows a partially exploded view of the present invention;
FIG.3 shows another partially exploded view of the present invention.

As illustrated in the figures: 1 denotes the massage head base; 11 denotes the connection columns; 111 denotes the positioning rods; 12 denotes the eccentric component; 2 denotes the massage head holders; 21 denotes the positioning holes; 22 denotes the fitting portions; 23 denotes the wire outlets; 24 denotes the accommodating columns; 241 denotes the positioning grooves; 3 denotes the silicone massage heads; 31 denotes the buffer spaces; 4 denotes the cloth covers; 5 denotes the inserts; 51 denotes the positioning columns; 6 denotes the heating members; 61 denotes the heating member electrical connection wires; 7 denotes the light boards; 71 denotes the light beads; 72 denotes the light board electrical connection wires.

### DETAILED DESCRIPTION OF THE INVENTION

To attain the above objects and effects, the features and functions of the technical solutions and structure adopted by the present invention will be described in detail in the preferred embodiments of the present invention with reference to the accompanying drawings, so as to aid thorough understanding.

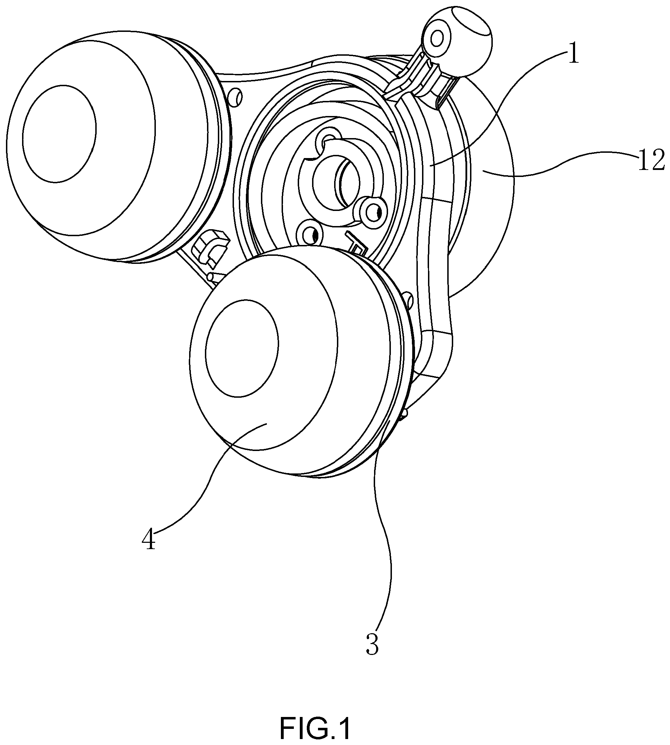

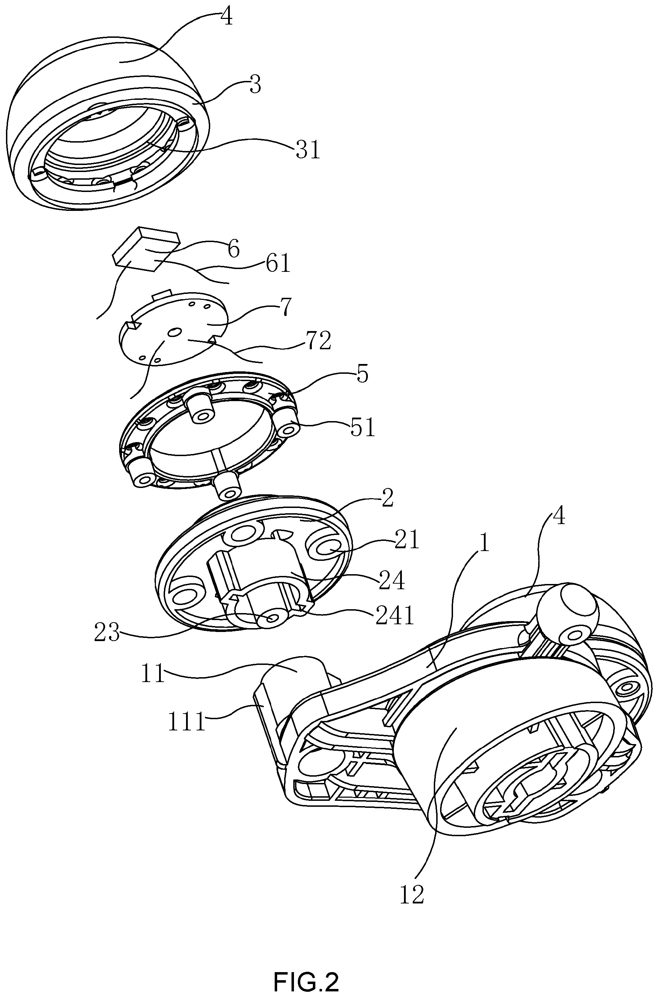

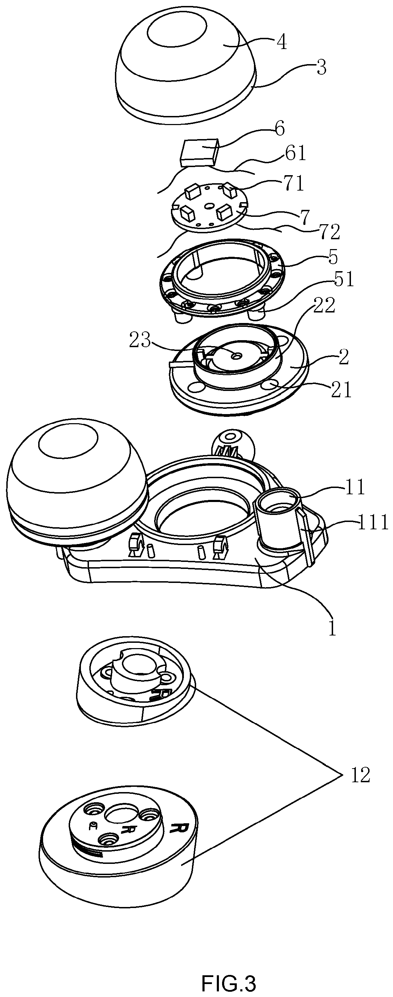

As illustrated in FIGS.1-3, the present invention discloses a massage head assembly comprising a massage head base 1, massage head holders 2 and silicone massage heads 3; each of the silicone massage heads 3 is fixedly disposed on the corresponding massage head holder 2; each of the massage head holders 2 is detachably disposed on the massage head base 1; each of the silicone massage heads 3 is of a hollow structure; each of the silicone massage heads 3 has an inner wall, and a buffer space 31 is formed between the inner wall and the corresponding massage head holder 2.

Therefore, the structure and design of the silicone massage heads 3 provide the present invention with a simple overall structure; due to the good flexibility of the silicone massage heads 3, and the buffer space 31 formed between the inner wall of each of the silicone massage heads 3 and the corresponding massage head holder 2 being capable of elastic deformation, the elasticity of the silicone massage heads 3 is increased, so that when the present invention is used to massage a human body, the silicone massage heads 3 can elastically deform to a larger degree, thereby providing a comfortable massage.

Furthermore, an outer surface of each of the silicone massage heads 3 is covered with a cloth cover 4; each of the cloth covers 4 can be an abrasion resistant cloth; for instance, each of the cloth covers 4 can be, but is not limited to, a nonwoven cloth or a woven cloth, which enables the present invention to perform a smooth and comfortable massage, and provides the silicone massage heads 3 with a better sliding effect.

Furthermore, the message head assembly of the present invention further comprises inserts 5 to facilitate fixed securement of the silicone massage heads 3 to the massage head holders 2; each of the inserts 5 is formed between a bottom part of each of the silicone massage heads 3 and a top surface of the corresponding massage head holder 2 by means of secondary plastic over-molding; the inserts 5 and the massage head holders 2 can be made of plastic materials.

Furthermore, a plurality of positioning columns 51 protrude from a bottom surface of each of the inserts 5 at intervals; a plurality of positioning holes 21 are provided on each of the massage head holders 2; each of the positioning columns 51 passes through the corresponding positioning hole 21 to facilitate positioning of the inserts 5 and the massage head holders 2 and forming of each of the inserts 5 between the bottom part of each of the silicone massage heads 3 and the top surface of the corresponding massage head holder 2 by means of secondary plastic over-molding.

Furthermore, a fitting portion 22 protrudes upwards from a middle part of each of the massage head holders 2; each of the inserts 5 is engaged with the corresponding fitting portion 22, so as to strengthen the connection between the inserts 5 and the massage head holders 2.

The inserts 5 can also be formed individually by injection molding; each of the cloth covers 4 covers an outer surface of the corresponding silicone massage head 3 to fix the corresponding insert 5 to the corresponding massage head holder 2; each of the silicone massage heads 3 is disposed on the corresponding insert 5 for placing into a silicone mold together to perform secondary plastic over-molding, so that each of the inserts 5 is fixedly disposed between the bottom part of the corresponding silicone massage head 3 and the top surface of the corresponding massage head holder 2.

Furthermore, to improve the massage effect of the present invention, the massage head assembly of the present invention further comprises heating members 6; each of the heating members 6 is fixedly disposed inside the corresponding silicone massage head 3; each of the heating members 6 can be formed inside the corresponding silicone massage head 3 by means of secondary plastic over-molding, or mounted independently inside the corresponding silicone massage head 3.

Furthermore, the massage head assembly of the present invention comprises light boards 7 each with a plurality of light beads 71; each of the heating members 6 is fixedly disposed on the corresponding light board 7; each of the light boards 7 is fixedly disposed on the corresponding massage head holder 2; each of the massage head holders 2 is provided with a wire outlet 23 for a light board electrical connection wire 72 of the corresponding light board 7 and a heating member electrical connection wire 61 of the corresponding heating member 6 to pass through; the light beads 71 are used to indicate the usage status of the present invention to users..

To facilitate assembly of the present invention, an accommodating column 24 protrudes downwards from the middle part of each of the massage head holders 2; the massage head base 1 is provided with connection columns 11; each of the connection columns 11 snaps into the corresponding accommodating column 24; at least two positioning grooves 241 recess into an inner wall of each of the accommodating columns 24 at intervals; at least two positioning rods 111 protrude from an outer wall of each of the connection columns 11 at intervals; each of the positioning rods 111 is slidably fitted inside the corresponding positioning groove 241, so as to facilitate fixed securement of the massage head holders 2 on the massage head base 1.

Furthermore, to improve the massage effect of the present invention, a middle part of the massage head base 1 is provided with an eccentric component 12, which enables the present invention to perform eccentric motions; in the present embodiment, the numbers of the positioning grooves 241 and the positioning rods 111 are both two; the numbers of the silicone massage heads 3, the massage head holders 2, the inserts 5, the light boards 7 and the heating members 6 are all two; the silicone massage heads 3 are disposed on two sides of the eccentric component 12 at intervals; the numbers of the connection columns 11 and the accommodating columns 24 are both two.

## Claims

1. A massage head assembly, comprising a massage head base (1), a massage head holder (2) and a silicone massage head (3); the silicone massage head (3) is fixedly disposed on the massage head holder (2); the massage head holder (2) is detachably disposed on the massage head base (1); the silicone massage head (3) is of a hollow structure; the silicone massage head (3) has an inner wall, and a buffer space (31) is formed between the inner wall and the massage head holder (2);
**characterized in that**:
the massage head assembly further comprises an insert (5); the insert (5) is formed between a bottom part of the silicone massage head (3) and a top surface of the massage head holder (2) by means of secondary plastic over-molding;
a plurality of positioning columns (51) protrude from a bottom surface of the insert (5) at intervals; a plurality of positioning holes (21) are provided on the massage head holder (2); each of the positioning columns (51) passes through the corresponding positioning hole (21);
a fitting portion (22) protrudes upwards from a middle part of the massage head holder (2); the insert is (5) engaged with the fitting portion (22).

2. The massage head assembly of claim 1, **characterized in that** an outer surface of the silicone massage head (3) is covered with a cloth cover (4).

3. The massage head assembly of claim 1, **characterized in that** the silicone massage head (3) assembly further comprises a heating member (6) ; the heating member (6) is fixedly disposed inside the silicone massage head (3).

4. The massage head assembly of claim 3, **characterized in that** the massage head assembly further comprises a light board (7) with a plurality of light beads (71); the heating member (6) is fixedly disposed on the light board (7); the light board (7) is fixedly disposed on the massage head holder (2); the massage head holder (2) is provided with a wire outlet (23) for a light board electrical connection wire (72) of the light board (7) and a heating member electrical connection wire (61) of the heating member (6) to pass through.

5. The massage head assembly of claim 1, **characterized in that** an accommodating column (24) protrudes downwards from a middle part of the massage head holder (2); the massage head base (1) is provided with a connection column (11); the connection column (11) snaps into the accommodating column (24).

6. The massage head assembly of claim 5, **characterized in that** at least two positioning grooves (241) recess into an inner wall of the accommodating column (24) at intervals; at least two positioning rods (111) protrude from an outer wall of the connection column (11) at intervals; each of the positioning rods (111) is slidably fitted inside the corresponding positioning groove (241).

7. The massage head assembly of claim 1, **characterized in that** a middle part of the massage head base (1) is provided with an eccentric component (12).

## Patentansprüche

1. - Massagekopfanordnung, umfassend eine Massagekopfbasis (1), einen Massagekopfhalter (2) und einen Silikonmassagekopf (3); wobei der Silikonmassagekopf (3) fest auf dem Massagekopfhalter (2) angeordnet ist; der Massagekopfhalter (2) abnehmbar an der Massagekopfbasis (1) angeordnet ist; der Silikonmassagekopf (3) von einer hohlen Struktur ist; der Silikonmassagekopf (3) eine Innenwand aufweist und ein Zwischenraum (31) zwischen der Innenwand und dem Massagekopfhalter (2) gebildet ist;
**dadurch gekennzeichnet, dass**:
die Massagekopfanordnung ferner einen Einsatz (5) umfasst; der Einsatz (5) durch sekundäres Umspritzen mit Kunststoff zwischen einem unteren Teil des Silikonmassagekopfs (3) und einer oberen Fläche des Massagekopfhalters (2) gebildet ist;
eine Vielzahl von Positionierungssäulen (51) in Abständen von einer unteren Fläche des Einsatzes (5) hervorstehen; eine Vielzahl von Positionierungslöchern (21) auf dem Massagekopfhalter (2) bereitgestellt sind; jede der Positionierungssäulen (51) durch das entsprechende Positionierungsloch (21) verläuft;
ein Passabschnitt (22) von einem mittleren Teil des Massagekopfhalters (2) nach oben hervorsteht; der Einsatz (5) mit dem Passabschnitt (22) in Eingriff ist.

2. - Massagekopanordnung nach Anspruch 1, **dadurch gekennzeichnet, dass** eine Außenfläche des Silikonmassagekopfs (3) mit einer Stoffhülle (4) bedeckt ist.

3. - Massagekopfanordnung nach Anspruch 1, **dadurch gekennzeichnet, dass** die Anordnung des Silikonmassagekopfs (3) ferner ein Heizelement (6) umfasst; das Heizelement (6) fest im Inneren des Silikonmassagekopfs (3) angeordnet ist.

4. - Massagekopfanordnung nach Anspruch 3, **dadurch gekennzeichnet, dass** die Massagekopfanordnung ferner eine Lichtplatte (7) mit einer Vielzahl von Lichtkugeln (71) umfasst; das Heizelement (6) fest auf der Lichtplatte (7) angeordnet ist; die Lichtplatte (7) fest auf dem Massagekopfhalter (2) angeordnet ist; der Massagekopfhalter (2) mit einem Drahtdurchlass (23) versehen ist, damit ein elektrischer Lichttafelverbindungsdraht (72) der Lichttafel (7) und ein elektrischer Heizelementverbindungsdraht (61) (6) hindurch verlaufen.

5. - Massagekopfanordnung nach Anspruch 1, **dadurch gekennzeichnet, dass** eine Aufnahmesäule (24) von einem mittleren Teil des Massagekopfhalters (2) nach unten hervorsteht; die Massagekopfbasis (1) mit einer Verbindungssäule (11) versehen ist; die Verbindungssäule (11) in die Aufnahmesäule (24) einrastet.

6. - Massagekopfanordnung nach Anspruch 5, **dadurch gekennzeichnet, dass** mindestens zwei Positionierungsnuten (241) in Abständen in einer Innenwand der Aufnahmesäule (24) ausgespart sind; mindestens zwei Positionierungsstangen (111) in Abständen von einer Außenwand der Verbindungssäule (11) hervorstehen; jede der Positionierungsstangen (111) gleitend in die entsprechende Positionierungsnut (241) eingesetzt ist.

7. - Massagekopfanordnung nach Anspruch 1, **dadurch gekennzeichnet, dass** ein mittlerer Teil der Massagekopfbasis (1) mit einer exzentrischen Komponente (12) versehen ist.

## Revendications

1. - Ensemble tête de massage, comprenant une base de tête de massage (1), un support de tête de massage (2) et une tête de massage en silicone (3) ; la tête de massage en silicone (3) est disposée de manière fixe sur le support de tête de massage (2) ; le support de tête de massage (2) est disposé de manière amovible sur la base de tête de massage (1) ; la tête de massage en silicone (3) a une structure creuse ; la tête de massage en silicone (3) a une paroi interne, et un espace tampon (31) est formé entre la paroi interne et le support de tête de massage (2) ;
**caractérisé par le fait que** l'ensemble tête de massage comprend en outre un insert (5) ; l'insert (5) est formé entre une partie inférieure de la tête de massage en silicone (3) et une surface supérieure du support de tête de massage (2) au moyen d'un surmoulage secondaire de matière plastique ;
une pluralité de colonnes de positionnement (51) font saillie à partir d'une surface inférieure de l'insert (5) à intervalles ; une pluralité de trous de positionnement (21) sont disposés sur le support de tête de massage (2) ; chacune des colonnes de positionnement (51) passe à travers le trou de positionnement correspondant (21) ;
une partie d'ajustement (22) fait saillie vers le haut à partir d'une partie centrale du support de tête de massage (2) ; l'insert (5) est engagé avec la partie d'ajustement (22).

2. - Ensemble tête de massage selon la revendication 1, **caractérisé par le fait qu'**une surface extérieure de la tête de massage en silicone (3) est recouverte d'une enveloppe en tissu (4).

3. - Ensemble tête de massage selon la revendication 1, **caractérisé par le fait que** l'ensemble de tête de massage en silicone (3) comprend en outre un élément chauffant (6) ; l'élément chauffant (6) est disposé de manière fixe à l'intérieur de la tête de massage en silicone (3).

4. - Ensemble tête de massage selon la revendication 3, **caractérisé par le fait que** l'ensemble tête de massage comprend en outre un panneau lumineux (7) avec une pluralité de perles lumineuses (71) ; l'élément chauffant (6) est disposé de manière fixe sur le panneau lumineux (7) ; le panneau lumineux (7) est disposé de manière fixe sur le support de tête de massage (2) ; le support de tête de massage (2) comporte une sortie de fil (23) pour le passage d'un fil de connexion électrique de panneau lumineux (72) du panneau lumineux (7) et d'un fil de connexion électrique d'élément chauffant (61) de l'élément chauffant (6).

5. - Ensemble tête de massage selon la revendication 1, **caractérisé par le fait qu'**une colonne de réception (24) fait saillie vers le bas à partir d'une partie centrale du support de tête de massage (2) ; la base de tête de massage (1) comporte une colonne de liaison (11) ; la colonne de liaison (11) s'enclenche dans la colonne de réception (24).

6. - Ensemble tête de massage selon la revendication 5, **caractérisé par le fait qu'**au moins deux rainures de positionnement (241) sont formées en creux à intervalles dans une paroi intérieure de la colonne de réception (24) ; au moins deux tiges de positionnement (111) font saillie à intervalles à partir d'une paroi extérieure de la colonne de liaison (11) ; chacune des tiges de positionnement (111) est emboîtée de manière coulissante à l'intérieur de la rainure de positionnement correspondante (241).

7. - Ensemble tête de massage selon la revendication 1, **caractérisé par le fait qu'**une partie centrale de la base de tête de massage (1) comporte un composant excentrique (12).
